Europäisches Patentamt

European Patent Office

Office européen des brevets

(19)

(11) Veröffentlichungsnummer: **0 012 986 B1**

(12) **EUROPÄISCHE PATENTSCHRIFT**

(45) Veröffentlichungstag der Patentschrift:
14.10.81

(21) Anmeldenummer: 79105255.8

(22) Anmeldetag: 18.12.79

(51) Int. Cl.³: **B 01 D 53/14**, B 01 D 53/34,
C 01 B 3/52, C 07 C 7/11, ·
C 07 C 9/04

(54) Verfahren zum Abtrennen und Gewinnen gasförmiger Komponenten aus einem Gasgemisch durch physikalische Wäsche.

(30) Priorität: 23.12.78 DE 2856078

(43) Veröffentlichungstag der Anmeldung:
09.07.80 Patentblatt 80/14

(45) Bekanntmachung des Hinweises auf die Patenterteilung:
14.10.81 Patentblatt 81/41

(84) Benannte Vertragsstaaten:
BE DE FR GB IT NL SE

(56) Entgegenhaltungen:
EP-A-0 004 043
FR-A-2 295 781
GB-A-855 543
US-A-3 030 182

(73) Patentinhaber: Linde Aktiengesellschaft,
Abraham-Lincoln-Strasse 21, D-6200 Wiesbaden (DE)

(72) Erfinder: Ranke, Gerhard, Feichtetstrasse 6,
D-8134 Pöcking (DE)
Erfinder: Weiss, Horst, Neufriedenheimer Strasse 37a,
D-8000 München 70 (DE)

(74) Vertreter: Schaefer, Gerhard, Dr., Linde
Aktiengesellschaft Zentrale Patentabteilung,
D-8023 Höllriegelskreuth (DE)

EP 0 012 986 B1

## Verfahren zum Abtrennen und Gewinnen gasförmiger Komponenten aus einem Gasgemisch durch physikalische Wäsche

Die Erfindung betrifft ein Verfahren zum Abtrennen und Gewinnen mindestens zweier gasförmiger Komponenten aus einem diese enthaltenden Gasgemisch durch Wäsche mit einer physikalisch wirkenden Waschflüssigkeit, gemäß dem Oberbegriff des Patentanspruchs 1.

Derartige Verfahren sind aus der DE-C-1 814 064 sowie der DE-B-2 548 700 vorbekannt.

Die DE-C-1 814 064 bezieht sich speziell auf die Entfernung von Kohlendioxid und Schwefelwasserstoff aus einem wasserstoffreichen Rohgas. Dieses wird in einer zweistufigen Wäsche zunächst im Gegenstrom zu bereits mit Kohlendioxid vorbeladenem Methanol vom Schwefelwasserstoff und anschließend mit vollständig regeneriertem Methanol vom Kohlendioxid befreit. Ein Teil des so in der zweiten Waschstufe mit Kohlendioxid vorbeladenen Methanols wird auf die erste Waschstufe aufgegeben und nach Aufnahme des Schwefelwasserstoffs als ein erster Waschflüssigkeitsstrom abgezogen. Der verbleibende Teil des aus der zweiten Waschstufe abgezogenen, mit Kohlendioxid vorbeladenen Methanols wird als ein zweiter Waschflüssigkeitsstrom aus der Wäsche abgeführt. Der erste Waschflüssigkeitsstrom wird unter Freisetzung einer gasförmigen, Kohlendioxid und Schwefelwasserstoff enthaltenden Fraktion entspannt. Diese wird im Gegenstrom mit dem ebenfalls entspannten zweiten Waschflüssigkeitsstrom vom Schwefelwasserstoff befreit, der die in Methanol im Vergleich zum Kohlendioxid besser lösliche Komponente darstellt. Aus dieser Behandlung resultiert somit ein erster, vorwiegend aus Kohlendioxid bestehender Produktgasstrom, der allerdings stark mit Stickstoff verunreinigt ist, da der erste Waschflüssigkeitsstrom nach der Entspannung und Freisetzung der gasförmigen Fraktion noch mit einem gasförmigen Stickstoffstrom gestrippt und der Strippgasstrom nach Aufnahme der zusätzlich freigesetzten Komponenten ebenfalls der Behandlung mit dem zweiten Waschflüssigkeitsstrom unterzogen wird, um mitausgestrippten Schwefelwasserstoff zurückzuwaschen. Die beiden Methanol-Waschflüssigkeitsströme werden schließlich vereinigt und einer Warmregenerierung zugeführt, die zur Entfernung und Gewinnung des aus Schwefelwasserstoff sowie restlichem Kohlendioxid bestehenden zweiten Produktgases führt.

Mit Hilfe dieses bekannten Verfahrens läßt sich zwar eine an Schwefelwasserstoff angereicherte gasförmige Produktfraktion gewinnen, der Rückgewinnungsgrad des Kohlendioxids ist jedoch äußerst mangelhaft. Die bei der Entspannung des aus mit Kohlendioxid vorbeladenem Methanol bestehenden zweiten Waschflüssigkeitsstromes freigesetzte gasförmige Kohlendioxidfraktion könnte zwar im Gegensatz zu dem in Figur 1 der DE-C-1 814 064 dargestellten Verfahrensablaufs getrennt, anstatt mit Strippgas vermischt gewonnen werden, die bei der Entspannung des zweiten, aus mit Kohlendioxid und Schwefelwasserstoff beladenem Methanol bestehenden Waschflüssigkeitsstromes freiwerdende Kohlendioxidfraktion wird jedoch beim bekannten Verfahren in jedem Fall mit Strippgas verunreinigt. Außerdem sind in der aus Schwefelwasserstoff und Kohlendioxid bestehenden, zweiten Produktgasfraktion noch relativ hohe Kohlendioxidanteile enthalten. Das Verfahren der DE-C-1 814 064 ist somit unter den Gesichtspunkten der Aufkonzentrierung des Schwefelwasserstoffs sowie der möglichst vollständigen Gewinnung des Kohlendioxids noch verbesserungsbedürftig.

Auch das zweite, aus der DE-B-2 548 700 vorbekannte Verfahren ist in der genannten Hinsicht noch nicht optimal, obwohl zusätzliche Verfahrensschritte vorgesehen sind. Diese bestehen darin, daß die bei der Entspannung des zweiten, lediglich mit Kohlendioxid vorbeladenen Waschflüssigkeitsstromes freigesetzte Kohlendioxidfraktion nicht mit Strippgas vermischt wird, und daß die Behandlung der gasförmigen Fraktion, die bei der Entspannung des ersten, Kohlendioxid und Schwefelwasserstoff enthaltenden Waschflüssigkeitsstromes freigesetzt wird, einerseits sowie die anschließende Strippung der beiden Waschflüssigkeitsströme andererseits in zwei getrennten Säulen durchgeführt werden. Infolgedessen wird nur mehr die beim Strippen freigesetzte Kohlendioxidfraktion mit Strippgas verunreinigt, während das beim Entspannen des ersten Waschflüssigkeitsstromes in die Gasphase übergehende Kohlendioxid nahezu rein gewonnen wird. Trotz dieser Maßnahme beträgt der Anteil des nahezu rein gewonnenen Kohlendioxids lediglich ca. 72%, während ca. 24% zusammen mit dem Strippgas abgeblasen werden und ca. 4% in der aufkonzentrierten Schwefelwasserstoff-Produktfraktion verbleiben.

Der Erfindung liegt die Aufgabe zugrunde, ein Verfahren der eingangs genannten Art bereitzustellen, das zu einer weiteren Verbesserung hinsichtlich der Rückgewinnung der abzutrennenden Komponenten führt, wobei das Verfahren gleichzeitig möglichst wirtschaftlich durchführbar sein soll.

Diese Aufgabe wird mit den im kennzeichnenden Teil des Patentanspruchs 1 genannten Maßnahmen gelöst.

Diese Maßnahmen führen zu einer erheblichen Verbesserung der Produktgasausbeute, sowohl hinsichtlich der Rückgewinnung der abgetrennten weniger gut löslichen Komponenten, im Falle der erwähnten vorbekannten Verfahren speziell des Kohlendioxids, als auch hinsichtlich der Aufkonzentration der besser löslichen Komponenten, im Falle der beiden vorbekannten Verfahren des Schwefelwasserstoffs, im zweiten Produktgas. Die weitere Entspannung der Waschflüssigkeitsströme führt zur Entgasung weiterer Teile der abgetrennten Komponenten, vorwiegend der weniger gut löslichen, zu einem gewissen Anteil aber auch der besser löslichen Komponenten. Letztere werden bei der Behandlung der freigesetzten gasförmigen Fraktion mit dem zweiten Waschflüssigkeitsstrom zurückgewaschen. Somit steigt die Ausbeute bezüglich der Gewinnung der weniger gut löslichen Komponenten, und gleichzeitig wird eine Aufkonzentrierung bezüglich der besser löslichen Komponenten in der Waschflüssigkeit erreicht. Die durch die weitere Entspannung verursachte Freisetzung der gasförmigen Fraktion führt zu einer Freisetzung der gasförmigen Fraktion führt zu einer Abkühlung der Waschflüssigkeit, der damit auf thermodynamisch günstige Weise ein Teil der in der Waschsäule entstandenen Lösungswärme wieder entzogen wird. Der zur Rückverdichtung der gasförmigen Fraktion benötigte Investitions- und Betriebsaufwand wird durch die erhöhte Produktausbeute mehr als wettgemacht.

In Weiterbildung der Erfindung ist vorgesehen, daß die Waschflüssigkeitsströme vor der weiteren Entspannung einer Zwischenentspannung unterzogen und rückverdichtet werden. Auch diese Zwischenentspannung führt zur Freisetzung einer gasförmigen Fraktion, die vorwiegend aus den weniger gut löslichen Komponenten besteht. Damit verbunden ist eine weitere Aufkonzentrierung der besser löslichen Komponenten in der Waschflüssigkeit. Die bei der Zwischenentspannung freigesetzte gasförmige Fraktion wird zweckmäßig rückverdichtet und ebenfalls der Behandlung mit dem zweiten Waschflüssigkeitsstrom unterzogen. Dies führt zu einer weiteren Steigerung der Ausbeute hinsichtlich der weniger gut löslichen Komponente.

Die Zwischenentspannung der Waschflüssigkeitsströme führt zu einer Abkühlung, vor allem aufgrund der dabei in die Gasphase übertretenden Fraktion. Die vorgesehene Rückverdichtung der Waschflüssigkeitsströme führt demgegenüber nur zu einer geringfügigen Erwärmung, da nur die flüssige Phase verdichtet wird. Daher ist vorgesehen, die Waschflüssigkeitsströme nach der Zwischenentspannung und Rückverdichtung zu erwärmen. Dies geschieht zweckmäßig im Gegenstrom zu abzukühlender, regenerierter sowie in der Waschsäule vorbeladener, Absorptionswärme enthaltender Waschflüssigkeit. Die Erwärmung führt zu einer weiteren Entgasung gelöster Komponenten. Dabei ist es wie in den oben behandelten Fällen zweckmäßig, daß die bei der Erwärmung freigesetzte gasförmige Fraktion ebenfalls der Behandlung mit dem zweiten Waschflüssigkeitsstrom unterzogen wird.

Durch die gemäß der Erfindung vorgesehene Behandlung von drei zusätzlichen gasförmigen Fraktionen mit dem zweiten Waschflüssigkeitsstrom wird somit gegenüber dem bekannten Verfahren eine erhebliche Steigerung der Ausbeute hinsichtlich der weniger gut löslichen Komponenten erzielt. Dies ist bereits der Fall, ohne daß wie etwa beim Verfahren der DE-C-1 814 064, unbedingt von einem Strippgas Gebrauch gemacht werden müßte.

Die Produktgasausbeute sowie die Aufkonzentrierung der besser löslichen Komponenten im zweiten Produktgas kann jedoch noch gesteigert werden, wenn die gemäß der Erfindung weiter entspannten Waschflüssigkeitsströme mit einem Inertgas gestrippt werden. Dabei soll auch hier das mit dabei freigesetzten gasförmigen Komponenten beladene Strippgas mit einem vom zweiten Waschflüssigkeitsstrom abgezweigten, ebenfalls entspannten Teilstrom behandelt und die gestrippten Waschflüssigkeitsströme anschließend der der Gewinnung des zweiten Produktgases dienenden Abtrennung zugeführt werden. Es erweist sich als besonders vorteilhaft, diese Strippung erst nach der erfindungsgemäßen Entspannung der Waschflüssigkeitsströme und gegebenenfalls der dieser vorausgehenden Zwischenentspannung und anschließenden Wiedererwärmung durchzuführen. Die Menge der nunmehr bei der Strippung freigesetzten gasförmigen Fraktion, deren Verunreinigung mit Strippgas nicht zu vermeiden ist, ist dann nämlich erheblich geringer als bei den bekannten Verfahren. Die Verluste der ins Restgas abzugebenden, weniger gut löslichen Komponenten können somit verringert werden. Falls die oben erwähnte Zwischenentspannung der Waschflüssigkeitsströme vorgenommen wird, ist vorgesehen, den zur Behandlung des beladenen Strippgases verwendeten, entspannten Teilstrom nach der besagten Behandlung mit den zwischenentspannten, noch nicht rückverdichteten Waschflüssigkeitsströmen zu vereinigen. Somit kann auch der für die Behandlung des beladenen Strippgases verwendete Teilstrom anschließend der weiteren Entspannung sowie gegebenenfalls der oben erwähnten Erwärmung unterzogen werden, wobei weitere gasförmige Anteile freigesetzt werden.

Da der Teilstrom vor der Behandlung des beladenen Strippgases entspannt werden muß, gehen auch dabei gewisse Anteile von der flüssigen in die gasförmige Phase über. Hierzu ist vorgesehen, die bei der Entspannung des Teilstromes freigesetzte gasförmige Fraktion ebenfalls rückzuverdichten und der Behandlung mit dem zweiten Waschflüssigkeitsstrom zu unterziehen. Dies liegt im Sinne der angestrebten, möglichst weitgehenden Rückgewinnung der weniger gut löslichen Komponenten mit dem ersten Produktgas.

Weiterhin bietet die Erfindung die Möglichkeit, auf einfache Weise das gewünschte Konzentrationsverhältnis der besser löslichen zu den weniger gut löslichen Komponenten im zweiten Produktgas einzustellen. Dies kann nämlich dadurch geschehen, daß der Enddruck der weiteren Entspannung der Waschflüssigkeitsströme in entsprechender Weise beeinflußt wird. Falls, wie dies im allgemeinen zweckmäßig sein wird, die bei der weiteren Entspannung freigesetzte gasförmige Fraktion mit einem Kompressor rückverdichtet wird, kann der Enddruck der weiteren Entspannung somit durch Regelung des Kompressor-Saugdruckes beeinflußt werden. Diese Maßnahme wirkt sich vor allem auf den Entgasungsgrad der weniger gut löslichen Komponenten aus, wodurch das Konzentrationsverhältnis im zweiten Produktgas in weiten Grenzen variierbar ist.

Im folgenden soll die Erfindung anhand von Ausführungsbeispielen näher erläutert werden. Die Ausführungsbeispiele beziehen sich auf die Abtrennung und Gewinnung von Kohlendioxid sowie Schwefelwasserstoff und Kohlenoxidsulfid aus einem Rohwasserstoffgemisch.

In den Abbildungen ist die zugehörige Verfahrensführung in schematischer Weise dargestellt.

Figur 1 zeigt eine Verfahrensvariante, die den Hauptgedanken der Erfindung verwirklicht.

Figur 2 zeigt eine Verfahrensvariante, die zusätzliche, im Rahmen der Erfindung liegende Verbesserungsmöglichkeiten aufweist.

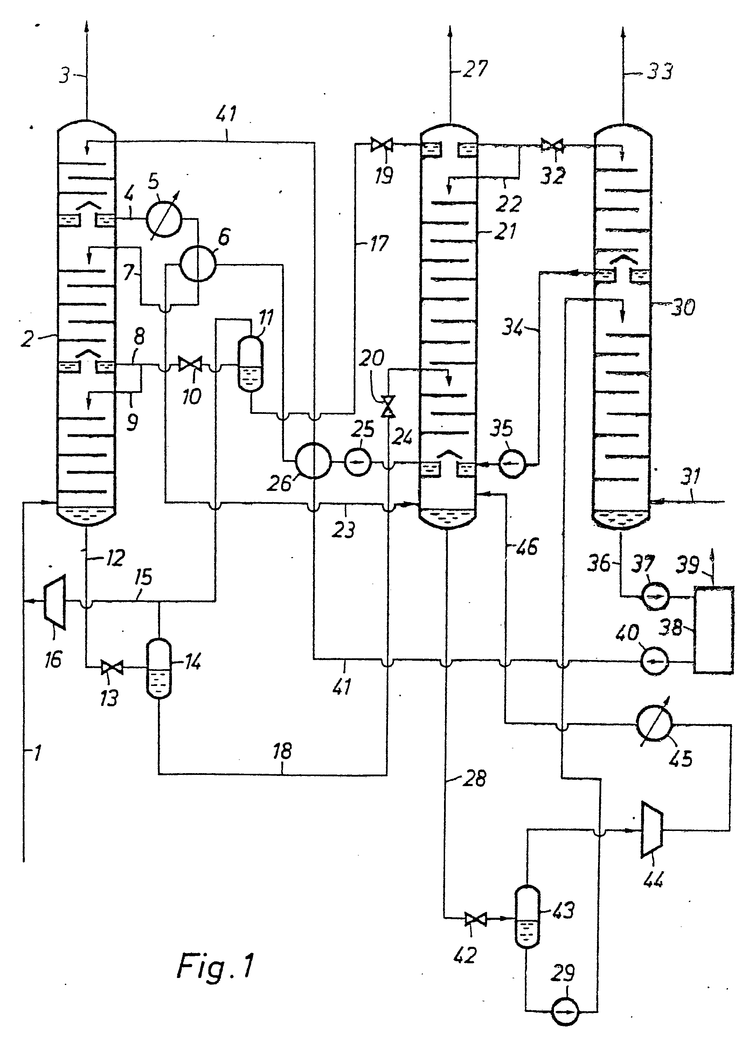

Gemäß Figur 1 werden durch eine Leitung 1 100 000 Nm$^3$/h eines Rohwasserstoff-Gasgemisches zugeführt, das 69 926 Nm$^3$/h Wasserstoff, 26 548 Nm$^3$/h Kohlendioxid, 1717 Nm$^3$/h Kohlenmonoxid, Argon und Methan, 1182 Nm$^3$/h Stickstoff sowie 627 Nm$^3$/h Schwefelwasserstoff und Kohlenoxidsulfid enthält. Dieses Gasgemisch gelangt nach Zuspeisung von 957 Nm$^3$/h eines verdichteten Rückführgases mit einem Druck von 75 bar in eine Waschsäule 2. Dort wird es mit Hilfe einer Waschflüssigkeit von Kohlendioxid, Schwefelwasserstoff und Kohlenoxidsulfid befreit, so daß am Kopf über eine Leitung 3 72 514 Nm$^3$/h eines mit Wasserstoff stark angereicherten Gasgemisches abgezogen werden, das nunmehr 69 677 Nm$^3$/h Wasserstoff, 1668 Nm$^3$/h Kohlenmonoxid, Argon und Methan sowie 1169 Nm$^3$/h Stickstoff enthält.

Auf den Säulenkopf werden über eine Leitung 41 als Waschflüssigkeit 100 t/h regenerierten Methanols mit einer Temperatur von 223 K aufgegeben. Dieses Waschmethanol nimmt im oberen Teil der Säule Kohlendioxid auf und erwärmt sich dabei. Zur Abführung von Absorptionswärme wird es über eine Leitung 4 abgezogen, in einem Kühler 5 mit Fremdkälte und in einem anschließenden Kühler 6 gegen kälteres Waschmethanol abgekühlt. Sodann wird es über eine Leitung 7 in den mittleren Teil der Säule zurückgegeben und schließlich über eine Leitung 8, mit Kohlendioxid nahezu gesättigt, abgezogen. Ein Teil, nämlich 45 t/h dieses mit Kohlendioxid vorbeladenen Waschmethanols, wird durch eine Leitung 9 auf den unteren Teil der Säule aufgegeben und nimmt dort sämtliche im Rohgasgemisch enthaltenen Anteile an Schwefelwasserstoff und Kohlenoxidsulfid auf. Vom Sumpf der Säule, in dem eine Temperatur von 266 K herrscht, werden über eine Leitung 12 45 t/h eines beladenen Waschmethanols abgezogen, das insgesamt 14 479 Nm$^3$/h gelöste Gase, d. h. Kohlendioxid, Schwefelwasserstoff und Kohlenoxidsulfid, mitführt.

Das vom Sumpf der Säule 2 abgezogene Waschmethanol wird anschließend in einem Entspannungsventil 13 auf einen Druck von 25 bar entspannt, wobei vor allem Wasserstoff entgast. Die gasförmigen Komponenten werden in einem Abscheider 14 abgetrennt und über eine Leitung 15 einem Kompressor 16 zugeführt, dort auf den Anfangsdruck von 75 bar rückverdichtet und mit dem Rohgasgemisch vereinigt. Vom Sumpf des Abscheiders 14 wird mit Kohlendioxid als der weniger gut löslichen Komponente sowie mit Schwefelwasserstoff und Kohlenoxidsulfid als den besser löslichen Komponenten beladenes Waschmethanol abgezogen, das nunmehr den ersten Waschflüssigkeitsstrom darstellt.

Der nicht über Leitung 9 in die Säule zurückgegebene Anteil des lediglich mit Kohlendioxid vorbeladenen Waschmethanols wird in einem Entspannungsventil 10 ebenfalls auf einen Druck von 25 bar entspannt. Die dabei entgasende Fraktion wird vom Kopf eines Abscheiders 11 abgeführt und mit der Kopffraktion des Abscheiders 14 vereinigt. Über Leitung 15 werden insgesamt 957 Nm$^3$/h eines Gasgemisches zurückgeführt, das 642 Nm$^3$/h Wasserstoff, 265 Nm$^3$/h Kohlendioxid, 30 Nm$^3$/h Kohlenmonoxid, Argon und Methan sowie 18 NM$^3$/h Stickstoff enthält. Die in den Abscheider 11 eingeführte Menge an Waschmethanol beträgt 55 t/h, unter Mitführung von 13 964 Nm$^3$/h gelöster Gase. Vom Sumpf des Abscheiders 11 wird ein vorwiegend mit Kohlendioxid beladenes Waschmethanol abgezogen, das den zweiten Waschflüssigkeitsstrom darstellt.

Der erste sowie der zweite Waschflüssigkeitsstrom werden über Leitungen 18 bzw. 17 Entspannungsventilen 20 bzw. 19 zugeführt und dort auf einen Druck von 3 bar entspannt. Beide werden in eine Anreicherungssäule 21 eingeführt, der erste Waschflüssigkeitsstrom in den Mittelteil, der zweite Waschflüssigkeitsstrom in den mit einem Abscheider versehenen Kopfteil. Bei der Entspannung in den Entspannungsventilen 20 bzw. 19 werden jeweils gasförmige Fraktionen freigesetzt, und zwar im Falle des zweiten Waschflüssigkeitsstromes eine vorwiegend aus Kohlendioxid bestehende Fraktion, die über Kopf der Säule abströmt, und im Falle des ersten Waschflüssigkeitsstromes eine sowohl Kohlendioxid als auch Schwefelwasserstoff und Kohlenoxidsulfid enthaltende Fraktion, die im Inneren der Säule nach oben steigt. Die Anteile an Schwefelwasserstoff und Kohlenoxidsulfid werden aus dieser aufsteigenden gasförmigen Fraktion durch einen Teil des zweiten Waschflüssigkeitsstromes zurückgewaschen, der aus dem Abscheider am Säulenkopf durch eine Leitung 22 in den oberen Teil der Anreicherungssäule 21 zurückgegeben wird. Dieser Teil des zweiten Waschflüssigkeitsstromes beträgt 35 t/h. Die über einem Kaminboden im unteren Teil der Anreicherungssäule 21 sich ansammelnden Waschflüssigkeitsströme werden über eine Leitung 24 abgezogen, mit Hilfe einer Flüssigkeitspumpe 25 durch Wärmeaustauscher 26 und 6 gefördert und anschließend über eine Leitung 23 in den untersten Teil der Anreicherungssäule, der lediglich als Abscheider fungiert, zurückgegeben. Dabei erwärmen sich die Waschflüssigkeitsströme im Wärmeaustausch mit durch eine Leitung 41 herangeführtem, regeneriertem Waschmethanol von 218 K auf 230 K sowie im Wärmeaustausch mit Absorptionswärme abgebenden, mit Kohlendioxid vorbeladenem Waschmethanol auf 240 K. Die durch die Erwärmung freigesetzte gasförmige Fraktion, die wiederum vor allem Kohlendioxid, aber auch Schwefelwasserstoff und Kohlenoxidsulfid enthält, strömt durch den Kaminboden der Anreicherungssäule 21 nach oben und wird dabei ebenfalls durch Gas-Flüssigkeits-Kontakt mit dem zweiten Waschflüssigkeitsstrom von den beiden schwefelhaltigen gasförmigen Komponenten befreit. Über dem Sumpf der Anreicherungssäule 21 werden durch eine Leitung 46 noch 5191 Nm³/h eines Gasgemisches eingeführt, das 4896 Nm³/h Kohlendioxid und 295 Nm³/h Schwefelwasserstoff und Kohlenoxidsulfid enthält. Vom Kopf der Anreicherungssäule 21 werden durch eine Leitung 27 32 316 Nm³/h eines ersten Produktgases abgezogen, das zum überwiegenden Teil, nämlich zu 23 000 Nm³/h, aus Kohlendioxid, zu 247 Nm³/h aus Wasserstoff, zu 49 Nm³/h aus Kohlenmonoxid, Argon und Methan sowie zu 20 Nm³/h aus Stickstoff besteht. Die Reinheit dieses gasförmigen Kohlendioxidprodukts beträgt somit ca. 98,7 Vol.-%. Dieses Produktgas fällt mit einer Temperatur von 224 K an.

Vom Sumpf der Anreicherungssäule 21 werden 100 t/h nunmehr an Schwefelwasserstoff und Kohlenoxidsulfid angereicherten Waschmethanols, bestehend aus den vereinigten ersten und zweiten Waschflüssigkeitsströmen, abgezogen. Dieses Waschmethanol führt in gelöster Form 7232 Nm³/h Kohlendioxid sowie 1025 Nm³/h Schwefelwasserstoff und Kohlenoxidsulfid mit. Es wird über eine Leitung 28 einem Entspannungsventil 42 zugeführt, dort auf einen Druck von 0,7 bar entspannt und in einen Abscheider 43 eingeführt. Die bei der Entspannung freigesetzte gasförmige Fraktion, zu 4896 Nm³/h aus Kohlendioxid und zu 295 Nm³/h aus Schwefelwasserstoff und Kohlenoxidsulfid bestehend, wird von einem Kompressor 44 angesaugt, dort auf etwa den Druck der Anreicherungssäule rückverdichtet, in einem Kühler 45 mittels Fremdkälte gekühlt und durch Leitung 46 in die Anreicherungssäule zurückgeführt, um dort der Behandlung mit dem zweiten Waschflüssigkeitsstrom unterzogen zu werden.

Vom Sumpf des Abscheiders 43 werden mittels einer Flüssigkeitspumpe 29 100 t/h Waschflüssigkeit abgezogen, die 2336 Nm³/h Kohlendioxid sowie 730 Nm³/h Schwefelwasserstoff und Kohlenoxidsulfid enthalten. Diese werden in eine Strippsäule 30 eingespeist und dort im unteren Teil im Gas-Flüssigkeits-Kontakt mit einem Strippgas geführt, das in einer Menge von 3000 Nm³/h durch eine Leitung 31 über dem Sumpf der Säule eingeführt wird. Bei dem Strippgas handelt es sich um Stickstoff. Während der Strippung gehen 1486 Nm³/h Kohlendioxid sowie 103 Nm³/h Schwefelwasserstoff und Kohlenoxidsulfid in die Gasphase über. Das so beladene Strippgas tritt durch einen etwa in der Mitte der Strippsäule 30 angeordneten Kaminboden in den oberen Teil der Säule ein, um dort von den beiden schwefelhaltigen Komponenten befreit zu werden. Dies geschieht mittels eines auf den Säulenkopf aufgegebenen, vom zweiten Waschflüssigkeitsstrom abgezweigten, in einem Entspannungsventil 32 auf einen Druck von 1,8 bar entspannten Teilstromes, der aus lediglich mit Kohlendioxid vorbeladenem Methanol besteht. Die Menge dieses Teilstromes beträgt 20 t/h. Der Teilstrom wird oberhalb des Kaminbodens, nunmehr 1878 Nm³/h Kohlendioxid sowie 103 Nm³/h Schwefelwasserstoff und Kohlenoxidsulfid mitführend, aus der Strippsäule abgezogen, mittels einer Flüssigkeitspumpe 35 auf den Druck der Anreicherungssäule 21 gefördert und in diese eingeführt. Vom Kopf der Strippsäule 30 werden über eine Leitung 33 5653 Nm³/h beladenen Strippgases abgeführt, das zu 2955 Nm³/h aus Stickstoff und zu 2698 Nm³/h aus Kohlendioxid besteht. Dieses beladene Strippgas fällt unter einem Druck von 1,8 bar an. Im unteren Teil der Strippsäule 30, wo der eigentliche Strippvorgang stattfindet, herrscht ein Druck von 2 bar. Vom Sumpf der Strippsäule werden über eine Leitung 36 100 t/h stark mit den schwefelhaltigen Komponenten angereicherten Waschmethode abgezogen, und zwar mit Hilfe einer Flüssigkeitspumpe 37. Dieses Waschmethanol, das 1517 Nm³/h gelöste Gase mitführt, wird in einer üblichen Warmregenerierung 38 vollständig von den gelösten Gasen befreit, so daß über eine Leitung 39 ein zweites Produktgas abgeführt werden kann, das aus 850 Nm³/h Kohlendioxid, 627 Nm³/h Schwefelwasserstoff und Kohlenoxidsulfid, 38 Nm³/h Stickstoff sowie 2 Nm³/h Wasserstoff besteht. Das vollständig regenerierte Waschmethanol wird mittels einer Flüssigkeitspumpe 40 über Leitung 41 auf den Kopf der Waschsäule 2 zurückgepumpt.

Eine Bilanz der Kohlendioxidausbeute zeigt im Falle des vorliegenden Ausführungsbeispiels folgendes Ergebnis: In dem über Leitung 27 abgezogenen ersten Produktgas werden 86,6% des mit dem Rohgas zugeführten Kohlendioxids zurückgewonnen, und zwar mit einer Reinheit von 98,7 Vol.-%. In dem über Leitung 33 abgezogenen beladenen Strippgas sind 10,2% des zugeführten Kohlendioxids enthalten, während in dem über Leitung 39 abströmenden zweiten Produktgas nur mehr 3,2% des Kohlendioxids verbleiben. Die entsprechenden Werte betragen beim bekannten Verfahren der DE-B-2 548 700 71,4% (98,4 Mol-%), ca. 24,3% sowie 4,23%. Hinsichtlich der auf das Kohlendioxid bezogenen Produktgasausbeute ist somit eine erhebliche Verbesserung festzustellen. Das gleiche gilt hinsichtlich der Anreicherung der schwefelhaltigen Komponenten Schwefelwasserstoff und Kohlenoxidsulfid im zweiten Produktgas: So beträgt beim Verfahren des obigen Ausführungsbeispiels das Konzentrationsverhältnis zwischen Kohlendioxid und den beiden schwefelhaltigen Komponenten im Rohgas 42,3, im zweiten Produktgas 1,356, was einer Aufkonzentrierung um einen Faktor 31,2 entspricht. Die entsprechenden Zahlenwerte für das Verfahren der DE-B-2 548 700 betragen 31,2 sowie 1,378 und 23,3. Auch bezüglich der Aufkonzentrierung im zweiten Produktgas bringt die Anwendung des Verfahrens gemäß der Erfindung demnach eine erhebliche Verbesserung.

Im obigen Ausführungsbeispiel wurde vorausgesetzt, daß eine ausreichende Menge Strippgas zur Verfügung steht. Die Erfindung ist jedoch auch dann anwendbar, wenn dies nicht der Fall ist. Zunächst ist die Konzentration der beiden schwefelhaltigen Komponenten in der Sumpfflüssigkeit des Abscheiders 43 zwar noch unbefriedigend, denn sie beträgt, bezogen auf die gesamte in der Flüssigkeit gelöste Menge an Kohlendioxid, Schwefelwasserstoff und Kohlenoxidsulfid, lediglich 21,1%. Eine derartige Konzentration reicht aber für die Weiterverarbeitung des zweiten Produktgases in einer Claus-Anlage nicht aus, da dort aus Wirtschaftlichkeitsgründen üblicherweise mindestens 25% Schwefelwasserstoffanteil gefordert sind. Die Konzentration der schwefelhaltigen Komponenten in der Sumpfflüssigkeit des Abscheiders 43 bzw. im zweiten Produktgas läßt sich jedoch in günstiger Weise dadurch beeinflussen, daß die Entspannung im Entspannungsventil 42 zu noch niedrigeren Drücken geführt wird. So reicht bereits eine Drucksenkung auf 0,48 bar aus, die Konzentration der schwefelhaltigen Komponenten auf 25,4% zu steigern. Eine derartige Drucksenkung führt nämlich nunmehr zur Entgasung von 5396 Nm³/h anstatt 4896 Nm³/h Kohlendioxid und 376 Nm³/h anstatt 295 Nm³/h Schwefelwasserstoff und Kohlenoxidsulfid. Der Anteil an gelöstem Kohlendioxid in der Sumpfflüssigkeit des Abscheiders 43 verringert sich damit auf 1836 Nm³/h, während der Anteil an schwefelhaltigen Komponenten auf 627 Nm³/h zurückgeht, einer Konzentration von 25,4% entsprechend. Dieser Effekt läßt sich durch weitere Drucksenkung noch steigern.

Die zuletzt geschilderte Verfahrensvariante weist mehrere Vorteile auf: es wird kein Strippgas benötigt, die Strippsäule entfällt, die Kohlendioxidausbeute wird wesentlich erhöht, da kein Kohlendioxid mit dem Strippgas verlorengeht, und der gesamte zweite Waschflüssigkeitsstrom kann auf den Kopf der Anreicherungssäule aufgegeben werden, d. h. die Anzahl der Austauscherböden bzw. die Menge der Füllkörperschüttung kann bei gleichbleibender Reinheit des vom Kopf der Anreicherungssäule über Leitung 27 abgezogenen ersten Produktgases reduziert werden.

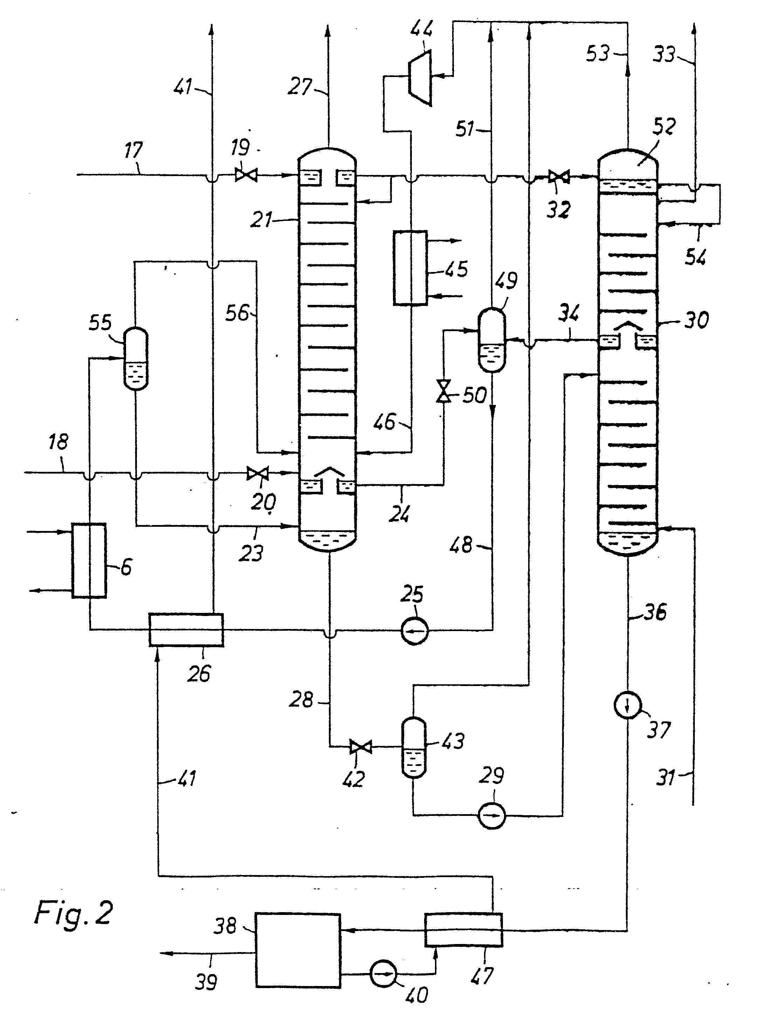

Das in Fig. 2 dargestellte weitere Ausführungsbeispiel der Erfindung zeigt zusätzliche Möglichkeiten zur Steigerung der Effektivität, die weiter oben bereits erwähnt und in einigen Unteransprüchen enthalten sind.

Fig. 2 ist insoweit an Fig. 1 angepaßt, als gleiche Vorrichtungteile mit gleichen Positionsnummern versehen sind. Weggelassen wurde in Fig. 2 der linke Teil der Fig. 1, insbesondere die Waschsäule 2 sowie die Abscheider 11 und 14 mit den zugehörigen Leitungen einschließlich der Leitungen 1 und 3 für das Rohgas bzw. das gereinigte Gas.

Die erwähnten zusätzlichen Möglichkeiten bestehen in der in einem Entspannungsventil 50 vorgenommenen Zwischenentspannung der vereinigten beiden Waschflüssigkeitsströme, die durch Leitung 24 aus der Anreicherungssäule 21 abgezogen werden, der in einem Abscheider 55 erfolgenden Freisetzung einer weiteren gasförmigen Fraktion aus der nach der Zwischenentspannung verbleibenden Waschflüssigkeit infolge vorhergehender Erwärmung sowie der gesonderten Gewinnung einer gasförmigen Kohlendioxidfraktion nach der Entspannung des auf die Strippsäule 30 aufzugebenden Teilstromes der Waschflüssigkeit, und zwar in einem Abscheider 52.

So können die über dem Kaminboden der Anreicherungssäule 21 durch Leitung 24 abgezogenen vereinigten Waschflüssigkeitsströme in einem Entspannungsventil 50 auf einen Zwischendruck gebracht und in den Abscheider 49 eingeführt werden. Die nach dieser Zwischenentspannung freigesetzte gasförmige Fraktion, die sowohl Kohlendioxid als auch geringere Anteile der beiden schwefelhaltigen Komponenten enthält, wird über eine Leitung 51 dem Kompressor 44 zugeführt, um anschließend in der Anreicherungssäule ebenfalls der Rückwaschung der schwefelhaltigen Komponenten unterzogen zu werden. Es ist zweckmäßig, auf den über dem Kaminboden der Strippsäule 30 herrschenden Druck zwischenzuentspannen, da die von dort über Leitung 34 abgezogene Waschflüssigkeit dann ohne Rückverdichtung mittels einer Flüssigkeitspumpe in den Abscheider 49 gefördert werden kann. Die Sumpfflüssigkeit des Abscheiders 49 wird dann mittels der Flüssigkeitspumpe 25 abgezogen und rückverdichtet sowie einer zweifachen Erwärmung in den Wärmeaustauschern 26 und 6 unterzogen, bevor sie durch Leitung 23 in den lediglich als Abscheider fungierenden unteren Teil der Anreicherungssäule 21 zurückgegeben wird. Die Erwärmung erfolgt im Wärmeaustauscher 26 gegen regeneriertes, abzukühlendes Waschmethanol und im Wärmeaustauscher 6 gegen mit Kohlendioxid vorbeladenes Waschmethanol aus dem oberen Teil der Waschsäule 2. Im Abscheider 55 wird eine gasförmige, Kohlendioxid, Schwefelwasserstoff sowie Kohlenoxidsulfid enthaltende Fraktion abgetrennt und über Leitung 56 in die Anreicherungssäule 21 eingeführt.

Der zum Rückwaschen von schwefelhaltigen Komponenten aus dem Strippgas dienende, im Entspannungsventil 32 entspannte Teilstrom des zweiten Waschflüssigkeitsstromes wird zunächst in einen auf die Strippsäule aufgesetzten Abscheider 52 eingespeist. Dort wird eine gasförmige, überwiegend Kohlendioxid enthaltende Fraktion abgetrennt und über eine Leitung 53 ebenfalls dem Kompressor 44 zugeführt. Hierdurch wird zusätzlich nahezu reines Kohlendioxid gewonnen. Es ist an sich nicht notwendig, diese Fraktion der Behandlung in der Anreicherungssäule zu unterziehen, da sie frei von Schwefelverbindungen ist. Es kann aber zweckmäßig sein, sie im Kompressor 44 mitzuverdichten und so auf den Druck des durch Leitung 27 abzuziehenden ersten Produktgases zu bringen. Der aufkonzentrierte Teilstrom wird über eine Leitung 54 aus dem Abscheider 52 abgezogen und in den oberen Abschnitt der Strippsäule 30 eingeführt, wo er zum Rückwaschen der schwefelhaltigen Komponenten aus dem Strippgas dient. Die in der Warmregenerierung 38 vollständig regenerierte Waschflüssigkeit wird üblicherweise in einen Wärmeaustauscher 47 gegen die durch Leitung 36 zuströmende, noch beladene Waschflüssigkeit einer ersten Abkühlung unterzogen.

Wie aus dem Vorstehenden zu entnehmen ist, kann das Verfahren gemäß der Erfindung bei der Abtrennung und Gewinnung saurer Gase aus diese enthaltenden Gasgemischen angewendet werden. Hierbei sind insbesondere Kohlendioxid als die weniger gut lösliche und Schwefelwasserstoff sowie Kohlenoxidsulfid als die besser löslichen Komponenten von Interesse, da die Abtrennung und Gewinnung derartiger Verunreinigungen aus Rohgasen ein besonders häufig auftretendes Problem darstellt. Solche Rohgase sind etwa Kohlevergasungsgase oder Gase, die durch partielle Oxidation flüssiger oder gasförmiger Kohlenwasserstoffe entstanden sind, insbesondere nach Konvertierung der Kohlenmonoxidanteile zu Kohlendioxid. Auf diesem Wege kann beispielsweise reiner Wasserstoff gewonnen werden. Die Erfindung ist jedoch keinesfalls hierauf beschränkt. So kann sie beispielsweise bei der Reinigung von Erdgas angewendet werden, wobei es ebenfalls um die Abtrennung von Kohlendioxid und Schwefelwasserstoff geht. Das Verhältnis von letzterem zu ersterem ist dabei im allgemeinen höher als bei den aus der Kohlenmonoxidkonvertierung hervorgehenden Gasen, und somit liegt das Schwergewicht der Verfahrensführung mehr bei der Schwefelwasserstoffaufkonzentrierung im zweiten Produktgas als bei der Gewinnung des Kohlendioxids als erstem Produktgas.

Die Erfindung ist ganz allgemein auf ein Gemisch mindestens dreier gasförmiger Komponenten anwendbar, wobei vorausgesetzt ist, daß eine Waschflüssigkeit verfügbar ist, die mindestens zwei der Komponenten mit ausreichender Selektivität gegenüber mindestens einer dritten absorbieren kann, und daß innerhalb der Gruppe dieser mindestens zwei absorbierten Komponenten wiederum deutlich unterschiedliche Absorbierbarkeiten vorliegen. Die Wirkung der erfindungsgemäßen Maßnahme liegt dabei in der Natur der physikalisch wirkenden Waschflüssigkeiten, so daß die Anwendbarkeit der Erfindung hinsichtlich der Art der physikalischen Waschflüssigkeit keinen Einschränkungen unterliegt. So können außer dem in den Ausführungsbeispielen genannten Methanol als physikalisch wirkende Waschflüssigkeiten beispielsweise Aceton, Dimethylformamid und/oder N-Methylpyrrolidon oder auch Mischungen von N-Methylpyrrolidon und Methanol verwendet werden, etwa zur Auswaschung und getrennten Gewinnung von Äthylen und Acetylen aus diese enthaltenden Gasgemischen. Die zuletzt genannten physikalisch wirkenden Waschflüssigkeiten besitzen alle eine gegenüber dem Äthylen stark ausgeprägte Selektivität für das Acetylen. Weiterhin könnte die Erfindung etwa bei der Druckölwäsche zur Gewinnung von Benzinkohlenwasserstoffen aus Erdgas oder Raffineriegasen angewendet werden, wobei die leichteren Benzinkohlenwasserstoffe als die weniger gut löslichen Komponenten von den schwereren Benzinkohlenwasserstoffen als den besser löslichen Komponenten abzutrennen sind.

**Patentansprüche**

1. Verfahren zum Abtrennen und Gewinnen mindestens zweier gasförmiger Komponenten aus einem diese enthaltenden Gasgemisch durch Wäsche mit einer physikalisch wirkenden Waschflüssigkeit, die für mindestens eine der abzutrennenden Komponenten ein besseres Lösungsvermögen als für mindestens eine andere dieser Komponenten besitzt, wobei aus der Wäsche ein erster, mit sämtlichen abzutrennenden Komponenten beladener und ein zweiter, lediglich Anteile der weniger gut löslichen Komponente oder Komponenten enthaltender Waschflüssigkeitsstrom abgezogen werden, eine gasförmige Fraktion durch Entspannen des ersten Waschflüssigkeitsstromes freigesetzt, durch Behandeln mit dem zweiten, ebenfalls entspannten Waschflüssigkeitsstrom von der oder den besser löslichen Komponenten befreit und als ein erstes Produktgas abgezogen wird, und die Waschflüssigkeitsströme schließlich unter Gewinnung eines zweiten Produktgases einer Abtrennung der besser löslichen sowie von Restanteilen der weniger gut löslichen Komponenten unterzogen werden, dadurch gekennzeichnet, daß die Waschflüssigkeitsströme vor der der Gewinnung des zweiten Produktgases dienenden Abtrennung weiter entspannt werden und die dabei freigesetzte gasförmige Fraktion rückverdichtet sowie ebenfalls der Behandlung mit dem zweiten Waschflüssigkeitsstrom unterzogen wird.

2. Verfahren nach Anspruch 1, dadurch gekennzeichnet, daß die Waschflüssigkeitsströme vor der weiteren Entspannung einer Zwischenentspannung unterzogen und rückverdichtet werden.

3. Verfahren nach Anspruch 2, dadurch gekennzeichnet, daß die bei der Zwischenentspannung freigesetzte gasförmige Fraktion rückverdichtet und ebenfalls der Behandlung mit dem zweiten Waschflüssigkeitsstrom unterzogen wird.

4. Verfahren nach den Ansprüchen 2 oder 3, dadurch gekennzeichnet, daß die Waschflüssigkeitsströme nach der Zwischenentspannung und Rückverdichtung erwärmt werden.

5. Verfahren nach Anspruch 4, dadurch gekennzeichnet, daß die bei der Erwärmung freigesetzte gasförmige Fraktion ebenfalls der Behandlung mit dem zweiten Waschflüssigkeitsstrom unterzogen wird.

6. Verfahren nach einem der Ansprüche 1 bis 5, dadurch gekennzeichnet, daß die weiter entspannten Waschflüssigkeitsströme mit einem Inertgas gestrippt werden, das mit dabei freigesetzten Komponenten beladene Strippgas mit einem vom zweiten Waschflüssigkeitsstrom abgezweigten, ebenfalls entspannten Teilstrom behandelt wird und die gestrippten Waschflüssigkeitsströme anschließend der der Gewinnung des zweiten Produktgases dienenden Abtrennung zugeführt werden.

7. Verfahren nach Anspruch 6, dadurch gekennzeichnet, daß der entspannte Teilstrom nach der Behandlung des beladenen Strippgases mit den zwischenentspannten, noch nicht rückverdichteten Waschflüssigkeitsströmen vereinigt wird.

8. Verfahren nach Anspruch 6 oder 7, dadurch gekennzeichnet, daß die bei der Entspannung des Teilstromes freigesetzte gasförmige Fraktion ebenfalls rückverdichtet und der Behandlung mit dem zweiten Waschflüssigkeitsstrom unterzogen wird.

9. Verfahren nach einem der Ansprüche 1 bis 8, dadurch gekennzeichnet, daß das gewünschte Konzentrationsverhältnis der besser löslichen zu den weniger gut löslichen Komponenten im zweiten Produktgas durch Beeinflussung des Enddruckes der weiteren Entspannung der Waschflüssigkeitsströme eingestellt wird.

10. Anwendung des Verfahrens nach den Ansprüchen 1 bis 9 zur Abtrennung und Gewinnung von sauren Gasen, insbesondere Kohlendioxid als der weniger gut löslichen und Schwefelwasserstoff und/oder Kohlenoxidsulfid als den besser löslichen Komponenten, aus diese enthaltenden Gasgemischen.

## Claims

1. A process for separating and obtaining at least two gaseous components from a gas mixture containing them by washing with a physically-acting washing liquid which possesses a better dissolving power with respect to at least one of the components which are to be separated than with respect to at least one other of said components, wherein a first stream of the washing liquid which contains all the components which are to be separated and a second stream of the washing liquid flow which contains only a part or parts of the less easily soluble component or components, are withdrawn from the washing step, a gaseous fraction is released by expanding the first washing liquid stream and is freed from the more easily soluble component or components by treatment with the second washing liquid stream, which has also been expanded, and withdrawn as a first product gas, and the more easily soluble components and residual portions of the less easily soluble components are separated from the washing liquid streams to provide a second product gas, characterised in that the washing liquid streams are further expanded prior to the separation step, in which the second product gas in produced, and the gaseous fraction which is thus released is recompressed and also subjected to treatment with the second washing liquid stream.

2. A process als claimed in Claim 1, characterised in that, prior to the further expansion, the washing liquid streams are subjected to an intermediate expansion and recompressed.

3. A process as claimed in Claim 2, characterised in that the gaseous fraction released during the intermediate expansion is recompressed and also subjected to treatment with the second washing liquid stream.

4. A process as claimed in Claim 2 or Claim 3, characterised in that the washing liquid streams are heated after the intermediate expansion and recompression.

5. A process as claimed in Claim 4, characterised in that the gaseous fraction released by the heating is also subjected to treatment with the second washing liquid stream.

6. A process as claimed in any one of Claims 1 to 5, characterised in that the further expanded washing liquid streams are stripped by means of an inert gas, the stripping gas containig the components which have thus been released, is treated with a similarly expanded substream which has been branched off from the second liquid stream, and the stripped washing liquid streams are subsequently fed to the separation stage which serves to produce the second product gas.

7. A process as claimed in Claim 6, characterised in that, after treatment with the charged stripping gas, the expanded sub-stream is combined with the intermediately expanded washing liquid streams, which have not yet been recompressed.

8. A process as claimed in Claim 6 or Claim 7, characterised in that the gaseous fraction which is released during the expansion of the sub-stream is also recompressed and subjected to treatment with the second washing liquid stream.

9. A process as claimed in any one of Claims 1 to 8, characterised in that the desired concentration ratio of the more easily soluble to the less easily soluble components in the second product gas is determined by influencing the final pressure of the further expansion of the washing liquid streams.

10. The use of the process as claimed in Claims 1 to 9 for the separation and obtaining of acid gases, in particular carbon dioxide as the less easily soluble, and hydrogen sulphide and/or carbon oxysulphide as the more easily soluble components, from gas mixtures which contain such components.

**Revendications**

1. Procédé pour la séparation et la récupération d'au moins deux constituants gazeux à partir d'un mélange gazeux les contenant par lavage avec un liquide de lavage à action physique qui possède pour au moins un des constituants à séparer un meilleur pouvoir solvant que pour au moins un autre de ces constituants, dans lequel on soutire du lavage un premier courant de liquide de lavage chargé de tous les constituants séparés et un deuxième courant ne contenant que des fractions du ou des constituants les moins solubles, dans lequel une fraction gazeuse, libérée par détente, du premier courant de liquide de lavage, débarrassé par traitement par le deuxième courant de liquide de lavage également détendu du ou des constituants plus solubles est soutirée comme premier produit gazeux et dans lequel les courants de liquide de lavage sont finalement soumis, après obtention d'un deuxième produit gazeux, à une séparation des constituants les plus solubles ainsi que des fractions restantes des constituants les moins solubles, caractérisé en ce que les courants de liquide de lavage sont détendus à nouveau avant la séparation servant à l'obtention du deuxième produit gazeux et que la fraction gazeuse alors libérée est condensée à nouveau et soumise également au traitement par le deuxième courant de liquide de lavage.

2. Procédé selon la revendication 1, caractérisé en ce que les courants de liquide de lavage sont soumis avant une nouvelle détente à une détente intermédiaire et à une recondensation.

3. Procédé selon la revendication 1, caractérisé en ce que la fraction gazeuse libérée lors de la détente intermédiaire est recondensée et également soumise au traitement par le deuxième courant de liquide de lavage.

4. Procédé selon la revendication 2 ou 3, caractérisé en ce que les courants de liquide de lavage sont chauffés après la détente intermédiaire et la recondensation.

5. Procédé selon la revendication 4, caractérisé par le fait que la fraction gazeuse libérée lors du chauffage est également soumise au traitement par le deuixième courant de liquide de lavage.

6. Procédé selon l'une quelconque des revendications 1 à 5, caractérisé en ce que les courants de liquide de lavage ayant subi une nouvelle détente sont entraînes par un gaz inerte, que le gaz d'entraînement chargé de composants libérés dans cette opération est traité par un courant partiel dérivé du deuxième courant de lavage ayant également subi une détente et que les courants de liquide de lavage ayant subi l'entraînement sont ensuite amenés vers la séparation servant à obtenir le deuxième produit gazeux.

7. Procédé selon la revendication 6, caractérisé en ce que le courant partiel ayant subi la détente est réuni après le traitement du gaz d'entraînement chargé avec les courants de liquide de lavage ayant subi la détente intermédiaire, non encore recondensés.

8. Procédé selon la revendication 6 ou 7, caractérisé en ce que la fraction gazeuse libérée lors de la détente du courant partiel est également recondensée et soumise au traitement par le deuxième courant de liquide de lavage.

9. Procédé selon l'une quelconque des revendications 1 à 8, caractérisé par le fait que l'on règle le rapport de concentration souhaité »constituants plus solubles/constituants moins solubles« dans le deuxième produit gazeux en influençant la pression finale de la détente supplémentaire des courants de gaz de lavage.

10. Utilisation du procédé selon l'une quelconque des revendications 1 à 9 pour la séparation et la récupération de gaz acides, notamment de gaz carbonique, comme constituants moins solubles et l'hydrogène sulfuré et/ou le sulfure de carbonyle comme constituants plus solubles, des mélanges gazeux les contenant.

Fig.1

Fig.2